# EUROPEAN PATENT SPECIFICATION

(11) **EP 4 308 218 B1**
(45) Date of publication and mention of the grant of the patent: **28.01.2026**
(21) Application number: 22770694.2
(22) Date of filing: 21.02.2022
(51) Int. Cl.: A61N 1/36, A61N 1/372, A61N 1/05, A61F 11/04, H04R 25/00

(54) **AUDITORY REHABILITATION FOR TELEPHONE USAGE**
AUDITORISCHE REHABILITATION FÜR TELEFONGEBRAUCH
RÉHABILITATION AUDITIVE POUR UTILISATION DE TÉLÉPHONE

(30) Priority: 18.03.2021 US 202163162637 P
(43) Date of publication of application: 24.01.2024
(73) Proprietor: Cochlear Limited, Macquarie University, New South Wales 2109 (AU)
(72) Inventor: OLIVER, Janette, Macquarie University, New South Wales 2109 (AU); VERMA, Rishubh, Macquarie University, New South Wales 2109 (AU); MCDONNELL, Viktorija, Macquarie University, New South Wales 2109 (AU)
(74) Representative: Grünecker Patent- und Rechtsanwälte PartG mbB
(86) International application number: PCT/IB2022/051523
(87) International publication number: WO 2022/195379

(56) References cited:
- EP-A1- 2 924 676
- CN-A- 101 751 799
- CN-A- 107 909 523
- KR-B1- 101 779 641
- US-A1- 2010 304 342
- US-A1- 2013 101 095
- US-A1- 2020 069 224

## Description

### BACKGROUND

### Field of the Invention

The present invention relates generally to auditory rehabilitation for recipients of hearing devices.

### Related Art

EP 2 924 676 A1 discloses a vocal training system comprising a training-control unit, an evaluation unit and a user-interface unit. The training-control unit generates an initial training-stimulus signal associated with a pre-stored template-sound information. The evaluation unit determines, from a training-activity, at least one error value of at least one error quantity indicative of a difference between the training-activity and template-sound information. The training-control unit receives the at least one error value and generates an adaptive response to the training-activity signal in the form of a modified training-stimulus that is modified in comparison with the last-generated training-stimulus in dependence on the at least one error value.
US 2020/069224 A1 discloses techniques for fitting and refitting of a DSP-based hearing assistance device. An audiologist user interface and a patient user interface include soundmaps and slidebars to control collections of hearing parameters within the DSP of the device. Based on cognitive testing and training of the patient, the range of adjustment allowed within at least the patient user interface is limited.
Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, *etc.),* pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

### SUMMARY

One or more non-transitory computer readable storage media according to the invention include the features defined in claim 1.

A system for developing cognitive resources of a recipient of hearing device for telephone usage according to the invention includes the features defined in claim 12.

Embodiments include the features defined in the dependent claims.

### BRIEF DESCRIPTION OF THE DRAWINGS

Embodiments of the present invention are described herein in conjunction with the accompanying drawings, in which:
FIG. 1A is a schematic diagram illustrating a cochlear implant system with which aspects of the techniques presented herein can be implemented;
FIG. 1B is a side view of a recipient wearing a sound processing unit of the cochlear implant system of FIG. 1A;
FIG. 1C is a schematic view of components of the cochlear implant system of FIG. 1A;
FIG. 1D is a block diagram of the cochlear implant system of FIG. 1A;
FIG. 2 is flowchart illustrating example phases of an auditory rehabilitation process for telephone usage, in accordance with certain embodiments presented herein;
FIG. 3 is a block diagram of an example computing device configured to implement aspects of an auditory rehabilitation process for telephone usage, in accordance with certain embodiments presented herein;
FIG. 4 is a flowchart of an example method, in accordance with embodiments presented herein;
FIG. 5 is a flowchart of another example method, in accordance with embodiments presented herein; and
FIG. 6 is a flowchart of another example method, in accordance with embodiments presented herein,

### DETAILED DESCRIPTION

Presented herein are auditory rehabilitation techniques facilitating independent usage of a telephone by a recipient of a hearing device. More specifically, the auditory rehabilitation techniques presented herein are configured to develop the cognitive resources of a recipient that are needed by the recipient to process, in real-time, stimulation signals having a degraded quality that is typical of telephonic signals, and in the absence of visual cues. In addition, the auditory rehabilitation techniques presented herein are configured to develop the cognitive resources of a recipient that are need to formulate, in real-time, spoken responses to the stimulation signals.

In general, and as described below, the auditory rehabilitation techniques presented herein use a multi-phase (multi-step) process to develop a recipient's cognitive resources in a manner that facilitates independent telephone usage. In one or more introductory phases, a recipient is introduced to a set of predetermined set of auditory targets. In one or more secondary phases, the auditory targets are used in a plurality of pre-recorded conversations delivered to the recipient, with increasing listening complexity, in order to develop auditory memory and introduce clarification and negotiation scenarios. In one or more additional phases, a plurality of virtual conversations are conducted with the recipient, with increasing complexity, in order to introduce and reinforce real-time telephonic interactions.

Merely for ease of description, the auditory rehabilitation techniques presented herein are primarily described with reference to a specific implantable medical device, namely a cochlear implant system comprising a cochlear implant. However, it is to be appreciated that the techniques presented herein may also be partially or fully implemented with other types of systems or devices, including other hearing device systems or hearing devices, such as hearing aids, middle ear auditory prostheses, bone conduction devices, direct acoustic stimulators, electro-acoustic prostheses, auditory brain stimulators, combinations or variations thereof, *etc.* The techniques presented herein may also be implemented by dedicated tinnitus therapy devices and tinnitus therapy device systems. In further embodiments, the presented herein may also be implemented by, or used in conjunction with, vestibular devices (e.g., vestibular implants), visual devices (i.e., bionic eyes), sensors, pacemakers, drug delivery systems, defibrillators, functional electrical stimulation devices, catheters, seizure devices (e.g., devices for monitoring and/or treating epileptic events), sleep apnea devices, electroporation devices, etc. The techniques presented herein may also be partially or fully implemented by consumer devices, such as tablet computers, mobile phones, wearable devices, etc.

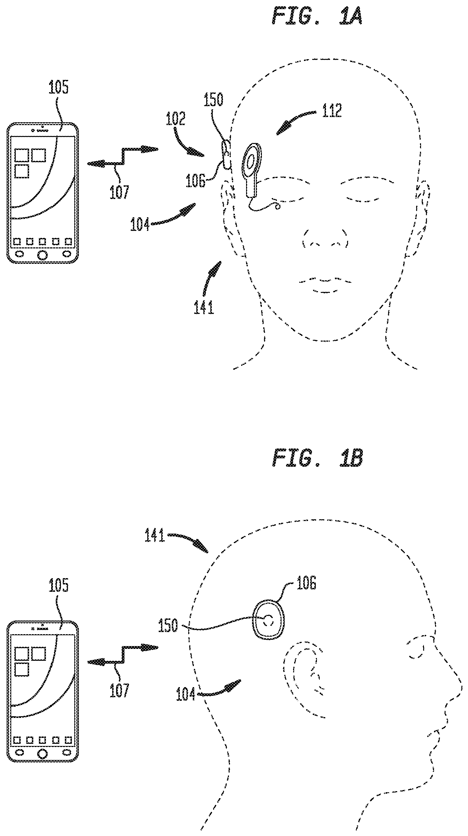

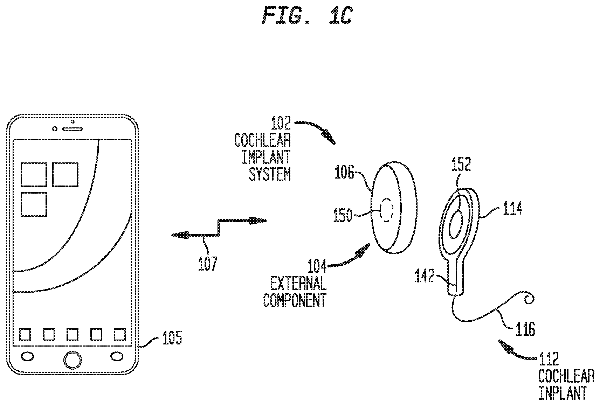

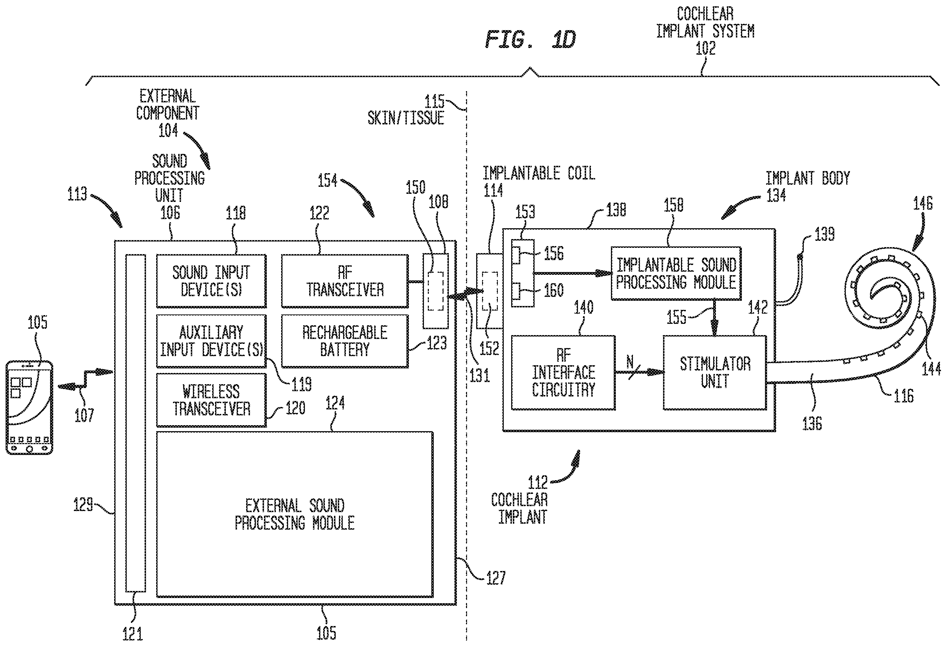

FIGs. 1A-1D illustrates an example cochlear implant system 102 with which aspects of the techniques presented herein can be implemented. The cochlear implant system 102 comprises an external component 104 and an implantable component 112. In the examples of FIGs. 1A-1D, the implantable component is sometimes referred to as a "cochlear implant." FIG. 1A illustrates the cochlear implant 112 implanted in the head 141 of a recipient, while FIG. 1B is a schematic drawing of the external component 104 worn on the head 141 of the recipient. FIG. 1C is another schematic view of the cochlear implant system 102, while FIG. 1D illustrates further details of the cochlear implant system 102. For ease of description, FIGs. 1A-1D will generally be described together.

Cochlear implant system 102 includes an external component 104 that is configured to be directly or indirectly attached to the body of the recipient and an implantable component 112 configured to be implanted in the recipient. In the examples of FIGs. 1A-1D, the external component 104 comprises a sound processing unit 106, while the cochlear implant 112 includes an internal coil 114, an implant body 134, and an elongate stimulating assembly 116 configured to be implanted in the recipient's cochlea.

In the example of FIGs. 1A-1D, the sound processing unit 106 is an off-the-ear (OTE) sound processing unit, sometimes referred to herein as an OTE component, that is configured to send data and power to the implantable component 112. In general, an OTE sound processing unit is a component having a generally cylindrically shaped housing 105 and which is configured to be magnetically coupled to the recipient's head (e.g., includes an integrated external magnet 150 configured to be magnetically coupled to an implantable magnet 152 in the implantable component 112). The OTE sound processing unit 106 also includes an integrated external (headpiece) coil 108 that is configured to be inductively coupled to the implantable coil 114.

It is to be appreciated that the OTE sound processing unit 106 is merely illustrative of the external devices that could operate with implantable component 112. For example, in alternative examples, the external component may comprise a behind-the-ear (BTE) sound processing unit or a micro-BTE sound processing unit and a separate external. In general, a BTE sound processing unit comprises a housing that is shaped to be worn on the outer ear of the recipient and is connected to the separate external coil assembly via a cable, where the external coil assembly is configured to be magnetically and inductively coupled to the implantable coil 114. It is also to be appreciated that alternative external components could be located in the recipient's ear canal, worn on the body, *etc.*

As noted above, the cochlear implant system 102 includes the sound processing unit 106 and the cochlear implant 112. However, as described further below, the cochlear implant 112 can operate independently from the sound processing unit 106, for at least a period, to stimulate the recipient. For example, the cochlear implant 112 can operate in a first general mode, sometimes referred to as an "external hearing mode," in which the sound processing unit 106 captures sound signals which are then used as the basis for delivering stimulation signals to the recipient. The cochlear implant 112 can also operate in a second general mode, sometimes referred as an "invisible hearing" mode, in which the sound processing unit 106 is unable to provide sound signals to the cochlear implant 112 (e.g., the sound processing unit 106 is not present, the sound processing unit 106 is powered-off, the sound processing unit 106 is malfunctioning, etc.). As such, in the invisible hearing mode, the cochlear implant 112 captures sound signals itself via implantable sound sensors and then uses those sound signals as the basis for delivering stimulation signals to the recipient. Further details regarding operation of the cochlear implant 112 in the external hearing mode are provided below, followed by details regarding operation of the cochlear implant 112 in the invisible hearing mode. It is to be appreciated that reference to the external hearing mode and the invisible hearing mode is merely illustrative and that the cochlear implant 112 could also operate in alternative modes.

In FIGs. 1A and 1C, the cochlear implant system 102 is shown with an external device 105, configured to implement aspects of the techniques presented. The external device 105 is a computing device, such as a computer (e.g., laptop, desktop, tablet), a mobile phone, remote control unit, *etc.* As described further below, the external device 105 comprises a telephone enhancement module that, as described further below, is configured to implement aspects of the auditory rehabilitation techniques presented herein for independent telephone usage. The external device 105 and the cochlear system 102 (e.g., OTE sound processing unit 106 or the cochlear implant 112) wirelessly communicate via a bi-directional communication link 107. The bi-directional communication link 107 may comprise, for example, a short-range communication, such as Bluetooth link, Bluetooth Low Energy (BLE) link, a proprietary link, etc.

Returning to the example of FIGs. 1A-1D, the OTE sound processing unit 106 comprises one or more input devices 113 that are configured to receive input signals (e.g., sound or data signals). The one or more input devices 113 include one or more sound input devices 118 (e.g., one or more external microphones, audio input ports, telecoils, *etc.),* one or more auxiliary input devices 119 (e.g., audio ports, such as a Direct Audio Input (DAI), data ports, such as a Universal Serial Bus (USB) port, cable port, *etc.),* and a wireless transmitter/receiver (transceiver) 120 (e.g., for communication with the external device 105). However, it is to be appreciated that one or more input devices 113 may include additional types of input devices and/or less input devices (e.g., the wireless short range radio transceiver 120 and/or one or more auxiliary input devices 119 could be omitted).

The OTE sound processing unit 106 also comprises the external coil 108, a charging coil 121, a closely-coupled transmitter/receiver (RF transceiver) 122, sometimes referred to as or radio-frequency (RF) transceiver 122, at least one rechargeable battery 123, and an external sound processing module 124. The external sound processing module 124 may comprise, for example, one or more processors and a memory device (memory) that includes sound processing logic. The memory device may comprise any one or more of: Non-Volatile Memory (NVM), Ferroelectric Random Access Memory (FRAM), read only memory (ROM), random access memory (RAM), magnetic disk storage media devices, optical storage media devices, flash memory devices, electrical, optical, or other physical/tangible memory storage devices. The one or more processors are, for example, microprocessors or microcontrollers that execute instructions for the sound processing logic stored in memory device.

The implantable component 112 comprises an implant body (main module) 134, a lead region 136, and the intra-cochlear stimulating assembly 116, all configured to be implanted under the skin/tissue (tissue) 115 of the recipient. The implant body 134 generally comprises a hermetically-sealed housing 138 in which RF interface circuitry 140 and a stimulator unit 142 are disposed. The implant body 134 also includes the internal/implantable coil 114 that is generally external to the housing 138, but which is connected to the transceiver 140 via a hermetic feedthrough (not shown in FIG. 1D).

As noted, stimulating assembly 116 is configured to be at least partially implanted in the recipient's cochlea. Stimulating assembly 116 includes a plurality of longitudinally spaced intra-cochlear electrical stimulating contacts (electrodes) 144 that collectively form a contact or electrode array 146 for delivery of electrical stimulation (current) to the recipient's cochlea.

Stimulating assembly 116 extends through an opening in the recipient's cochlea (e.g., cochleostomy, the round window, *etc.)* and has a proximal end connected to stimulator unit 142 via lead region 136 and a hermetic feedthrough (not shown in FIG. 1D). Lead region 136 includes a plurality of conductors (wires) that electrically couple the electrodes 144 to the stimulator unit 142. The implantable component 112 also includes an electrode outside of the cochlea, sometimes referred to as the extra-cochlear electrode (ECE) 139.

As noted, the cochlear implant system 102 includes the external coil 108 and the implantable coil 114. The external magnet 152 is fixed relative to the external coil 108 and the implantable magnet 152 is fixed relative to the implantable coil 114. The magnets fixed relative to the external coil 108 and the implantable coil 114 facilitate the operational alignment of the external coil 108 with the implantable coil 114. This operational alignment of the coils enables the external component 104 to transmit data and power to the implantable component 112 via a closely-coupled wireless RF link 131 formed between the external coil 108 with the implantable coil 114. In certain examples, the closely-coupled wireless link 131 is a radio frequency (RF) link. However, various other types of energy transfer, such as infrared (IR), electromagnetic, capacitive and inductive transfer, may be used to transfer the power and/or data from an external component to an implantable component and, as such, FIG. 1D illustrates only one example arrangement.

As noted above, sound processing unit 106 includes the external sound processing module 124. The external sound processing module 124 is configured to convert received input signals (received at one or more of the input devices 113) into output signals for use in stimulating a first ear of a recipient (i.e., the external sound processing module 124 is configured to perform sound processing on input signals received at the sound processing unit 106). Stated differently, the one or more processors in the external sound processing module 124 are configured to execute sound processing logic in memory to convert the received input signals into output signals that represent electrical stimulation for delivery to the recipient.

As noted, FIG. 1D illustrates an embodiment in which the external sound processing module 124 in the sound processing unit 106 generates the output signals. In an alternative embodiment, the sound processing unit 106 can send less processed information (e.g., audio data) to the implantable component 112 and the sound processing operations (e.g., conversion of sounds to output signals) can be performed by a processor within the implantable component 112.

Returning to the specific example of FIG. 1D, the output signals are provided to the RF transceiver 122, which transcutaneously transfers the output signals (e.g., in an encoded manner) to the implantable component 112 via external coil 108 and implantable coil 114. That is, the output signals are received at the RF interface circuitry 140 via implantable coil 114 and provided to the stimulator unit 142. The stimulator unit 142 is configured to utilize the output signals to generate electrical stimulation signals (e.g., current signals) for delivery to the recipient's cochlea. In this way, cochlear implant system 102 electrically stimulates the recipient's auditory nerve cells, bypassing absent or defective hair cells that normally transduce acoustic vibrations into neural activity, in a manner that causes the recipient to perceive one or more components of the received sound signals.

As detailed above, in the external hearing mode the cochlear implant 112 receives processed sound signals from the sound processing unit 106. However, in the invisible hearing mode, the cochlear implant 112 is configured to capture and process sound signals for use in electrically stimulating the recipient's auditory nerve cells. In particular, as shown in FIG. 1D, the cochlear implant 112 includes a plurality of implantable sound sensors 153 and an implantable sound processing module 158. Similar to the external sound processing module 124, the implantable sound processing module 158 may comprise, for example, one or more processors and a memory device (memory) that includes sound processing logic. The memory device may comprise any one or more of: Non-Volatile Memory (NVM), Ferroelectric Random Access Memory (FRAM), read only memory (ROM), random access memory (RAM), magnetic disk storage media devices, optical storage media devices, flash memory devices, electrical, optical, or other physical/tangible memory storage devices. The one or more processors are, for example, microprocessors or microcontrollers that execute instructions for the sound processing logic stored in memory device.

In the invisible hearing mode, the implantable sound sensors 153 are configured to detect/capture signals (e.g., acoustic sound signals, vibrations, etc.), which are provided to the implantable sound processing module 158. The implantable sound processing module 158 is configured to convert received input signals (received at one or more of the implantable sound sensors 153) into output signals for use in stimulating the first ear of a recipient (i.e., the processing module 158 is configured to perform sound processing operations). Stated differently, the one or more processors in implantable sound processing module 158 are configured to execute sound processing logic in memory to convert the received input signals into output signals 155 that are provided to the stimulator unit 142. The stimulator unit 142 is configured to utilize the output signals 155 to generate electrical stimulation signals (e.g., current signals) for delivery to the recipient's cochlea, thereby bypassing the absent or defective hair cells that normally transduce acoustic vibrations into neural activity.

It is to be appreciated that the above description of the so-called external hearing mode and the so-called invisible hearing mode are merely illustrative and that the cochlear implant system 102 could operate differently in different embodiments. For example, in one alternative implementation of the external hearing mode, the cochlear implant 112 could use signals captured by the sound input devices 118 and the implantable sound sensors 153 in generating stimulation signals for delivery to the recipient.

The present inventors have recognized specific aspects of the auditory rehabilitation pathway for a recipient of a hearing device, and in particular the recipient of a cochlear implant, can be difficult with different technologies. In particular, independent use of a telephone is characteristically difficult for many hearing device recipients, particular those with sensorineural hearing loss treated with a cochlear implant or other electrically-stimulating hearing device. However, the ability to use the telephone independently correlates strongly with perceived quality of life and is one of the most widespread expectations held by cochlear implant candidates.

For many cochlear implant recipients, a telephone conversation is perceived as a stressful event, where the cochlear implant recipient often requires another person's nearby presence in order to have quick assistance in case of, for example, communication failures, an ability to understand conversational context, etc. This inability to use a telephone independently can reduce social connectivity, which in turn can result in an associated decreased quality of life, a depression state, and/or cognitive decline. Challenges experienced with telephone usage may be due to factors such as stimulus type (degraded digital signal quality resulting from telephonic transmission, compared to high fidelity signals captured from live voice), unfamiliar topics and unknown speakers, previous negative experience on the telephone, absence of visual cues such as eye contact, facial expression, lip movement and body language and even background noise.

For individuals with normal hearing, listening is generally effortless when conditions are ideal. Even under challenging listening situations, a normal auditory system can routinely extract the meanings of signals that are degraded, embedded in noise or lacking visual cues. In contrast, for individuals with hearing loss, listening is generally effortful even when conditions are ideal. The challenge in situations where the auditory-only signal is degraded or embedded in noise is greatly magnified due, at least in part, to the reduction in signal precision/granularity associated with electrical hearing relative to normal hearing (e.g., thousands of hair cell receptors versus only ten to twenty two electrodes). The absence of visual cues makes the listening task even more difficult (requires more effort). The use of linguistic and acoustic context can dramatically enhance the ability of a listener to recognize and understand what they have heard and respond appropriately. This, however, requires the engagement of executive processes that regulate, control, and manage other cognitive processes, such as attention and working memory. Increased cognitive resources are needed, elevating the cognitive load and requiring greater listening effort and motivation to engage meaningfully.

There are two general processes involved in auditory perception, referred to as "bottom-up processing" and "top-down processing." Bottom-up processing relates to processing of information as it is received (i.e., in real-time), and refers to the way the cognitive resources are built up from small pieces of sensory information. Top-down processing, however, refers to perception that is driven by cognition and how the brain considers context and applies what the brain knows and what the brain is expecting to perceive and fills in any gaps.

When processing written information, an individual can control the rate of input by, for example, reading more slowly. However, in hearing and listening tasks, perceptual interpretation and judgement must be made in real-time. This increases the dependence on contextual information. Therefore, speech perception requires the rapid integration of bottom-up processing with top-down processing.

In addition, meaningful participation in a conversation further requires the recipient to respond appropriately and in real-time. Such communication involves the working memory system, which becomes increasingly engaged in understanding speech with degraded input. In general, working memory refers to the ability to simultaneous store and process received information.

Furthermore, the more resources that are allocated to recovering a degraded input signal, the fewer resources, or cognitive spare capacity (CSC), remain for higher-level processing (e.g., in the sense of processing the meaning rather than just peripheral detection) of the speech input. A lack of cognitive spare capacity can make listening very tiring and effortful. This listening fatigue further impacts the cognitive load experienced by the individual.

A major challenge for adult aural rehabilitation is that the processes involved in listening are primarily cognitive, yet listening is perceived behaviorally. This has caused intervention approaches to look at cognition and behavior associated with listening as separate phenomena and therefore managed in separate silos. However, listening and communication are not experienced as different things by the individual in daily life.

As such, the auditory rehabilitation techniques presented herein utilize a combined auditory-cognitive training approach, where cognitive enhancement is embedded within auditory tasks, which is most likely to offer generalized benefits to the real-world listening and communication abilities of adults with hearing loss. The auditory rehabilitation techniques presented therefore provide a framework for recipients to build and practice real time listening comprehension and spoken response skills directly related to meaningful telephone usage in a safe practice environment, such as on their mobile device.

In other words, presented herein are techniques to increase the cognitive resources specifically associated with telephone usage (i.e., the cognitive resources needed by a hearing device recipient to successfully use a telephone independently). For a matter of convenience, the specific set of cognitive resources that a recipient needs to successfully use a telephone independently are sometimes referred to herein as "telephone cognitive resources." These telephone cognitive resources, once developed, allow a recipient to successfully process "telephone data" in real-time, while simultaneously form spoken responses thereto. Telephone data is generally comprised of signals delivered to the recipient via a hearing device, where the data has a degraded quality associated with telephone transmission/communication (e.g., received at hearing device from a telephone). As noted elsewhere herein, due to the fact that the telephone data is received from a telephone, the telephone data is associated with a degraded digital signal quality (in contrast to higher fidelity live voice), an absence of visual cues, and often associated with unfamiliar topics, unknown speakers, and/or background noise.

It is to be appreciated that the "telephone cognitive resources" included cognitive resources that can be used in other contexts and for other cognitive processing. However, as noted, the techniques presented herein are configured to specifically improve the recipient's cognitive abilities in a manner that allows the recipient to successfully process telephone data, in real-time and formulated appropriate responses in real-time, as needed, thereby enabling independent telephone usage by the recipient. As described further below, the techniques presented herein utilize an interactive training method, including virtual agents and/or conversational artificial intelligence (AI), along with services supported by advanced wireless streaming and connectivity, to increase the cognitive resources of a recipient associated with telephone usage (telephone cognitive resources). As a result, the techniques presented herein enable a hearing device recipient to, over time, use a telephone with reduced cognitive effort, which in turn facilitates independent and regular telephone usage for the recipient. As noted, independent and regular telephone usage for everyday communication and social interaction allows the hearing device recipient to, for example, increase their independence and self-confidence.

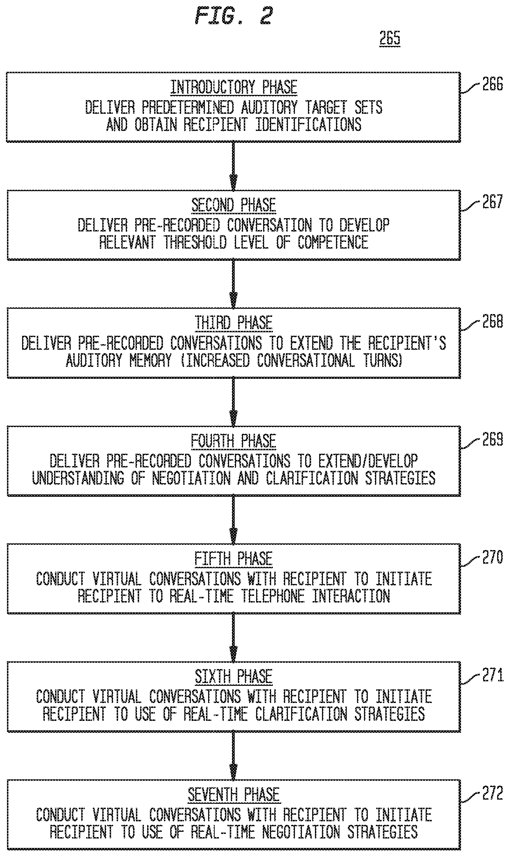

FIG. 2 is general flow diagram illustrating training stages/phases in accordance with an example method 265 presented herein. It is to be appreciated that the specific training stages and training stage structures, as well as the order thereof, shown in FIG. 2 is merely illustrative and that the techniques presented herein can be implemented with different numbers of stages in various combinations.

In general, the content modules shown in FIG. 2 are designed to complement training to increase bottom-up processing of the signal with more ecologically valid training to boost top-down information processing based on knowledge. Training in the use of various types of context builds on linguistic and world knowledge with the aim being to focus attention to facilitate comprehension, memory, and speed of information processing.

As described further below, the training method 265 is delivered across several levels/phases starting from introductory warm up phases based on bottom up processing and identification of basic auditory targets. The conversational levels start with passive listening comprehension and progress through to advanced interactive conversations requiring both bottom up and top down information processing and spoken language responses in real time. The method 265 can be implemented on a computing device, such as a mobile phone (smartphone), tablet computer, laptop computer, or other device capability of replicating a telephone call quality. For ease of description, method 265 is described with reference to cochlear implant system 102 and external device 105, where the external device 105 is a mobile phone.

As shown in FIG. 2, method 265 begins at a first or "introductory" level/phase 266 where the recipient listens a closed set of predetermined auditory targets for telephone usage. The predetermined auditory targets, which are sometimes referred to as "auditory targets," relate to information/data that is likely to be encountered by the recipient while using a telephone. The auditory targets can include, for example, transactional information (e.g., numbers, etc.) information related to social planning (e.g., dates, names, locations, times, etc.), and/or other types of information. That is, the predetermined auditory targets are not a selection of random words, but instead are predetermined lists designed to introduce the recipient to specific types of information likely to be encountered when using a telephone.

At 266, the mobile phone 105 emits at least one (e.g., one or a plurality of) acoustic sound signal representing at least one auditory target via an integrated receiver (not shown in FIGs. 1A-1D) and the at least one acoustic sound signal target is received at the cochlear implant system 102 (e.g., via the sound input devices 118 and/or the implantable sensors 153). The at least one acoustic sound signal is processed by the cochlear implant system 102 (e.g., external sound processing module 124 and/or implantable sound processing module 158) and then delivered to the recipient as electrical signals via the electrodes 144, to evoke perception of the at least one auditory target. Following delivery of the at least one auditory target to the recipient (as electrical signals), the recipient uses a user interface (e.g., touch screen) of the mobile phone 105 to identify what she heard. This process may be repeated for each of the predetermined auditory targets or groups of predetermined auditory targets.

In certain embodiments, following delivery of each of the at least one auditory target, the user interface of the mobile phone 105 can be configured to provide the recipient with a series of text or pictorial responses from which the recipient can select in order to identify what she heard. The mobile phone 105 can provide feedback to the recipient indicating whether or not she correctly perceived the predetermined auditory targets. In other embodiments, the user can enter text at the mobile phone 105 to identify what she heard, while in still other embodiments the mobile phone 105 can be configured to receive a spoken/verbalized indication from the recipient (e.g., use a speech-to-text function). It is to be appreciated that these manners in which the recipient indication is received are merely illustrative and that other techniques can be used in alternative embodiments.

In certain embodiments, the method 265 is self-guided and the recipient can proceed to the next stage, or repeat the introductory or first phase 266, or portions thereof, and/or at her discretion. In other embodiments, a monitoring engine (e.g., artificial intelligence (AI) engine) can monitor the method 265 and recommend to the recipient when she should proceed to the to the next stage and/or whether the recipient should repeat all or portions of the introductory phase 266.

Once the recipient is comfortable with the auditory targets (and/or the monitoring engine recommends that the recipient proceed), the method 265 proceeds to a second phase 267. During the second phase 267, the recipient listens to pre-recorded conversations between two voices, the content of which is relatively simple and easy to comprehend. The conversations are provided to the recipient via the cochlear implant system 102, in the manner as described above. Following each conversation, the recipient is then asked to respond to a question about the content of the conversation by selecting from a list of possible answers displayed at a user interface of the mobile phone 105. In general, the conversations provided during the second phase 267 are configured to develop a "relevant threshold level of competence" in the recipient as related to passively listening for meaning and responding to questions of elementary comprehension. That is, as used herein, a "relevant threshold level of competence" refers to the development of a minimum ability in the recipient to passively listen to, and comprehend, elementary or fundamental aspects of a short conversation.

Again, as noted, method 265 may be self-guided where the recipient can progress to a next stage at her discretion and/or method 265 can be controlled with monitoring engine to recommend to the recipient when she should proceed to the to the next stage and/or whether the recipient should repeat all or portions of a present stage.

In certain embodiments, the recipient cannot progress from the second phase 267 until the recipient has demonstrated the relevant threshold level of competence. Development of the relevant threshold level of competence could be evidenced, for example, by the recipient correctly answering a certain number or percentage of context questions.

Upon completion of the second phase, the method 265 proceeds to a third phase 268. During the third phase 268, the recipient again listens to pre-recorded conversations between two voices, but the conversations are more difficult for the recipient to follow (e.g., increased listening complexity). In particular, the conversations in the third phase 268 include more conversational turns, relative to the conversations in the second phase 267, and are designed to extend the recipient's auditory memory.

Again, the pre-recorded conversations are provided to the recipient via the cochlear implant system 102, in the manner as described above. Following each conversation, the recipient is then asked to respond to one or more questions about the content of the conversation by, for example, selecting from a list of possible answers displayed at a user interface of the mobile phone 105. As above, method 265 may be self-guided where the recipient can progress to a next phase at her discretion and/or method 265 can be controlled with monitoring engine to recommend to the recipient when she should proceed to the to the next stage and/or whether the recipient should repeat all or portions of a present stage.

In certain embodiments, the recipient cannot progress from the third phase 268 until the recipient has demonstrated an acceptable level of competence with the increased conversation turns. The acceptable level of competence with the increased conversation turns could be evidenced, for example, by the recipient correctly answering a certain number or percentage of context questions.

Upon completion of the third phase, the method 265 proceeds to a fourth phase 269. During the fourth phase 269, the recipient again listens to pre-recorded conversations between two voices, but the conversations are more difficult for the recipient to follow (e.g., even greater listening complexity). In particular, the conversations in the fourth phase 269 include even more conversational turns, relative to the conversations in the third phase 268, and but also add in negotiation and clarification scenarios in the content. As used herein, clarification scenarios are conversation portions in which one of the two voices clarifies or corrects statements made by the other voice (e.g., "Not five guests, only four guests," and so on). As used herein, negotiation scenarios are conversation portions in which the two voices perform a real-time negotiation (e.g., First Voice: We don't have tables available at 6:00 PM. I can give you a table at 5:30 PM."; Second Voice: "No, are there tables available at 6:30 or 7:00 PM?" and so on).

Again, the pre-recorded conversations in the fourth phase 269 are provided to the recipient via the cochlear implant system 102, in the manner as described above. Following each conversation, the recipient is then asked to respond to one or more questions about the content of the conversation by, for example, selecting from a list of possible answers displayed at a user interface of the mobile phone 105. Relative to the second and third phases, the questions in the fourth phase 269 are more difficult and can relate to, for example, the negotiation and clarification scenarios presented during the conversations. In certain embodiments, the recipient cannot progress from the fourth phase 269 until the recipient has demonstrated an acceptable level of competence with the negotiation and clarification scenarios. The acceptable level of competence with negotiation and clarification scenarios could be evidenced, for example, by the recipient correctly answering a certain number or percentage of context questions.

Upon completion of the fourth phase 269, the method 265 proceeds to a fifth phase 270. The fifth phase 270 is different from prior phases in that this first phase requires real-time interaction between the recipient and a virtual assistant (bot). That is, the fifth phase 270 newly introduces the need for the recipient to actively respond by providing her own spoken answers, in a manner that is similar to a real-world interactive conversation. In this fifth phase 270, the complexity of the conversation is relatively straightforward and may be less complex than the conversations in, for example, the third phase 268 and/or the fourth phase 269. However, the increased cognitive difficulty associated with the fifth phase 270 arises due to the recipient having to interactively respond, instead of passively listening as in the earlier phases. In general, the fifth phase 270 requires the recipient to answer common questions encountered in typical telephone conversations using known contexts.

In the fifth phase 270, and subsequent phases described below, the techniques presented herein can use speech recognition with natural language processing/understanding to receive spoken/verbalized responses from the recipient during the virtual conversation. In addition, voice and/or text interfaces and/or conversational artificial intelligence can be used to create bots or virtual agents to dynamically generate conversation portions/segments that are delivered to the recipient (as acoustic sound signals received via the cochlear implant system 102). That is, the external device 105 is configured (e.g., via conversational artificial intelligence) to tailor the conversation to the responses of the recipient, thereby allowing interactive telephone experiences that do not feel overly scripted. Features like error handling can ensure conversations remain on-track, while integration with a content management backend (CMS) backend will allow for personalization for a given recipient.

The dynamically generation of conversation portions enable the recipient to develop the cognitive resources needed to listen to a conversation, as well as the cognitive resources associated with real-time communication skills to deal independently with everyday transactional telephone tasks, such as, booking tickets or restaurants, ordering take-out food, making appointments and social plans.

Returning to the example of FIG. 2, upon completion of the fifth phase 270, the method 265 proceeds to a sixth phase 271. Similar to the fifth phase 270, the sixth phase 271 also requires real-time interaction between the recipient and a virtual assistant (bot). However, the sixth phase 271 again increases the listening complexity by asking the recipient to listen to longer and/or more complex sentences, with additional conversation turns, in the virtual conversation. The sixth phase 271 can use known contexts, but also introduces clarification scenarios (e.g., increased response complexity), which are conversation portions that require the recipient to employ/perform clarification strategies in real-time. That is, the virtual conversation in the sixth phase 271 require the recipient to add clarifications as part of her response (e.g., "Not five guests, only four guests," and so on).

Upon completion of the sixth phase 270, the method 265 proceeds to a seventh phase 272. Similar to the sixth phase 271, the seventh phase 272 also requires real-time interaction between the recipient and a virtual assistant (bot). However, the seventh phase 272 again increases the complexity by asking the recipient to listen to still longer and/or more complex sentences, with additional conversation turns, in the virtual conversation. The seventh phase 272 also adds in "sabotage" or "negotiation" scenarios (e.g., even greater increased response complexity), which are conversation portions that require the recipient to employ negotiation strategies in real-time. That is, the virtual conversation in the seventh phase 272 requires the recipient to perform negotiation (e.g., as part of her spoken response) (e.g., Virtual Assistant: We don't have tables available at 6:00 PM. I can give you a table at 5:30 PM." Recipient: "No, are there tables available at 6:30 or 7:00 PM?" and so on).

As noted above, the pre-recorded conversations and virtual conversations are provided to the recipient via the cochlear implant system 102, in the manner as described above. In the virtual conservations, the recipient speaks her responses, which are captured by the mobile phone 105. Also as noted above, method 265 may be self-guided where the recipient can progress to a next phase at her discretion and/or method 265 can be controlled with monitoring engine to recommend to the recipient when she should proceed to the to the next stage and/or whether the recipient should repeat all or portions of a present stage.

In the example of FIG. 2, the software at the external device 105 is configured to control volume, word rate and pitch and modify a variety of spoken language characteristics such as pauses and the degree of emphasis on given words. Such flexibility allows the techniques presented herein to build increasing levels of listening and response complexity including emotional context of speech for the hearing device recipient. Other features that may be used to add complexity or functionality include the ability to add sound files to play during conversation, such as playing environmental background noise or simulated telephone signal degradation/distortion, can be used in any or all of the different phases.

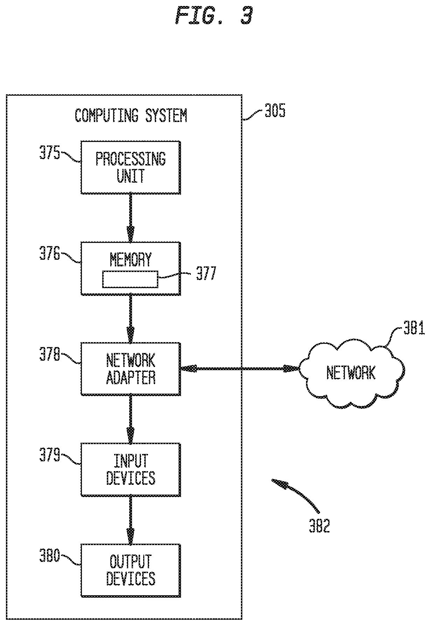

FIG. 3 illustrates an example arrangement for a suitable external device (computing device) configured to implement aspects of the techniques presented herein. Computing devices, environments, or configurations that can be suitable for use with examples described herein include, but are not limited to, personal computers, server computers, hand-held devices, laptop devices, multiprocessor systems, microprocessor-based systems, programmable consumer electronics (e.g., smart phones), network PCs, minicomputers, mainframe computers, tablet computers (tablets), distributed computing environments that include any of the above systems or devices, and the like. The external devices presented herein can be a single virtual or physical device operating in a networked environment over communication links to one or more remote devices. The remote device can be a hearing device or hearing device system (e.g., cochlear implant 112 or cochlear implant system 105 of FIG. 1D) an auditory prosthesis), a personal computer, a server, a router, a network personal computer, a peer device or other common network node. For ease of description, the external device shown in FIG. 3 is referred to as external device 305, and can represent a basic arrangement for external device 105 of FIGs. 1A-1D.

In its most basic configuration, the external device 305 includes at least one processing unit 375 and memory 376. The processing unit 375 includes one or more hardware or software processors (e.g., Central Processing Units) that can obtain and execute instructions. The processing unit 375 can communicate with and control the performance of other components of the computing system 305.

The memory 376 is one or more software or hardware-based computer-readable storage media operable to store information accessible by the processing unit 375. The memory 376 can store, among other things, instructions executable by the processing unit 375 to implement applications or cause performance of operations described herein, as well as other data. The memory 376 can be volatile memory (e.g., RAM), non-volatile memory (e.g., ROM), or combinations thereof. The memory 376 can include transitory memory or non-transitory memory. The memory 376 can also include one or more removable or non-removable storage devices. In examples, the memory 376 can include RAM, ROM, EEPROM (Electronically-Erasable Programmable Read-Only Memory), flash memory, optical disc storage, magnetic storage, solid state storage, or any other memory media usable to store information for later access. In examples, the memory 376 encompasses a modulated data signal (e.g., a signal that has one or more of its characteristics set or changed in such a manner as to encode information in the signal), such as a carrier wave or other transport mechanism and includes any information delivery media. By way of example, and not limitation, the memory 376 can include wired media such as a wired network or direct-wired connection, and wireless media such as acoustic, RF, infrared and other wireless media or combinations thereof. In certain embodiments, the memory 376 comprises telephone enhancement logic 377 that, when executed, enables the processing unit 375 to perform aspects of the techniques presented.

In the illustrated example, the external device 305 further includes a network adapter 378, one or more input devices 379, and one or more output devices 380. The one or more input devices 379 and the one or more output devices 380 are sometimes collectively referred to herein as a user interface 382 and can comprise the same or different components. The external device 305 can include other components, such as a system bus, component interfaces, a graphics system, a power source (e.g., a battery), among other components.

The network adapter 378 is a component of the external device 305 that provides network access (e.g., access to at least one network 381). The network adapter 378 can provide wired or wireless network access and can support one or more of a variety of communication technologies and protocols, such as ETHERNET, cellular, BLUETOOTH, near-field communication, Radio Frequency (RF), infrared (IR), among others. The network adapter 378 can include one or more antennas and associated components configured for wireless communication according to one or more wireless communication technologies and protocols.

The one or more input devices 379 are devices over which the external device 305 receives input from a user, such as a recipient during method 265 described above. The one or more input devices 379 can include physically-actuatable user-interface elements (e.g., buttons, switches, or dials), touch screens, keyboards, mice, pens, and voice/sound input devices, among others input devices.

The one or more output devices 380 are devices by which the external device 305 is able to provide output to a user. The output devices 380 can include, displays, receivers, and/or speakers, among other output devices.

It is to be appreciated that the arrangement for external device 305 shown in FIG. 3 is merely illustrative and that aspects of the techniques presented herein may be implemented at a number of different types of systems/devices. For example, the external device 305 could be a laptop computer, tablet computer, mobile phone, surgical system, etc.

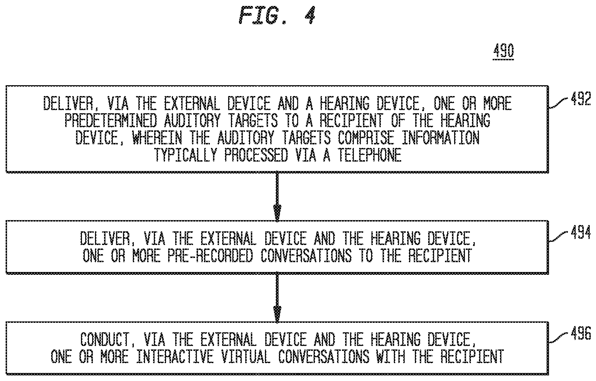

FIG. 4 is a flowchart of an example method 490, in accordance with certain embodiments presented herein. Method 490 begins at 492 where an external device and a hearing device deliver one or more predetermined auditory targets to a recipient of the hearing device, wherein the auditory targets comprise information typically processed via a telephone. At 494, the external device and the hearing device deliver one or more pre-recorded conversations to the recipient. At 496, the external device conducts, via the hearing device, one or more interactive virtual conversations with the recipient.

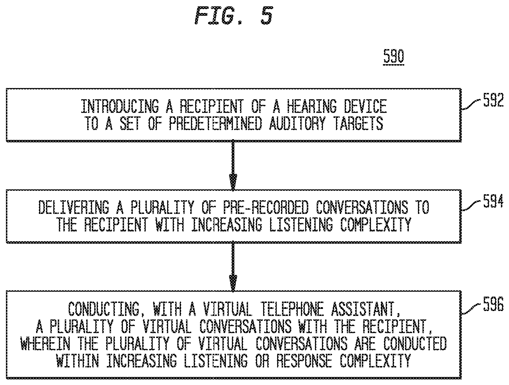

FIG. 5 is a flowchart of an example method 590, in accordance with certain embodiments presented herein. Method 590 begins at 592 where a recipient of a hearing device is introduced to a set of predetermined auditory targets. At 594, an external device delivers a plurality of pre-recorded conversations to the recipient with increasing listening complexity. At 596, the external device conducts, with a virtual telephone assistant, a plurality of virtual conversations with the recipient, wherein the plurality of virtual conversations are conducted within increasing listening or response complexity.

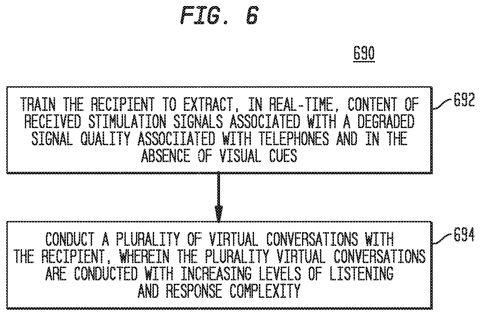

FIG. 6 is a flowchart of an example method 690, in accordance with certain embodiments presented herein. Method 690 begins at 692 where an external device trains a recipient of a hearing device to extract, in real-time, content of received stimulation signals associated with a degraded signal quality associated with telephones and in the absence of visual cues. At 694, the external device conducts a plurality of virtual conversations with the recipient, wherein the plurality virtual conversations are conducted with increasing levels of listening and response complexity.

## Claims

1. One or more non-transitory computer readable storage media comprising instructions that, when executed by a processor of an external device, cause the processor to:
deliver (266, 492), via the external device and a hearing device, one or more predetermined auditory targets to a recipient of the hearing device, wherein the one or more predetermined auditory targets comprise information typically processed via a telephone;
deliver (267, 268, 269, 494), via the external device and the hearing device, one or more pre-recorded conversations to the recipient; and
conduct (270, 271, 272, 496), via the external device and the hearing device, one or more interactive virtual conversations with the recipient.

2. The one or more non-transitory computer readable storage media of claim 1, wherein the instructions operable to deliver (266, 492) the one or more predetermined auditory targets to the recipient comprise instructions operable to:
generate one or more sound signals at the external device, wherein each of the one or more sound signals represents a predetermined auditory target and wherein each of the one or more sound signals is delivered to the recipient via the hearing device used by the recipient; and
following delivery of each of the one or more sound signals to the recipient, receive an indication of the recipient's perception of each of the one or more sound signals, in particular receive the indication of the recipient's perception via a user interface of the external device or receive a spoken indication of the recipient's perception via a microphone of the external device.

3. The one or more non-transitory computer readable storage media of claim 1 or 2, wherein the instructions operable to deliver (267, 268, 269, 404) each of the one or more pre-recorded conversations to the recipient via the hearing device comprise instructions operable to:
generate a sequence of acoustic sound signals at the external device, wherein each acoustic sound signal represents an associated pre-recorded conversation and wherein the sequence of acoustic sound signals is delivered to the recipient via the hearing device.

4. The one or more non-transitory computer readable storage media of claim 1 or 2, further comprising instructions operable to:
following delivery of each of the one or more pre-recorded conversations to the recipient via the hearing device, query the recipient with one or more questions regarding a content of a preceding pre-recorded conversation, wherein each of the one or more questions is displayed at a user interface of the external device; and
receive, from the recipient, a response to each of the one or more questions, in particular receive a user input via one or more input devices of the user interface or receive a spoken response to each of the one or more questions.

5. The one or more non-transitory computer readable storage media of claim 1 or 2, wherein the instructions operable to deliver (267, 268, 269, 494) the one or more pre-recorded conversations to the recipient via the hearing device comprise instructions operable to:
deliver (267) a first set of pre-recorded conversations to the recipient via the hearing device, wherein each of the pre-recorded conversations in the first set of pre-recorded conversations has approximately a first number of conservational turns and a first content level configured to develop a relevant threshold level of competence; and
following delivery (267) of the first set of pre-recorded conversations, deliver (268) a second set of pre-recorded conversations to the recipient via the hearing device, wherein each of the pre-recorded conversations in the second set of pre-recorded conversations has at least a second number of conservational turns that are greater than the first number of conversational turns.

6. The one or more non-transitory computer readable storage media of claim 5, wherein the instructions operable to deliver (267, 268, 269, 494) the one or more pre-recorded conversations to the recipient via the hearing device further comprise instructions operable to at least one of:
- following delivery (268) of the second set of pre-recorded conversations, deliver a third set of pre-recorded conversations to the recipient via the hearing device, wherein each of the pre-recorded conversations in the third set of pre-recorded conversations has at least a third number of conservational turns that are greater than the second number of conversational turns; and
- following delivery (268) of the second set of pre-recorded conversations, deliver (269) a third set of pre-recorded conversations to the recipient via the hearing device, wherein each of the pre-recorded conversations in the third set of pre-recorded conversations includes at least one of a negotiation scenario and a clarification scenario.

7. The one or more non-transitory computer readable storage media of claim 1 or 2, wherein the instructions operable to conduct (270, 271, 272, 496) each of the one or more interactive virtual conversations with the recipient comprise instructions operable to conduct one or more target virtual conversations, and wherein conducting at least one of the one or more target virtual conversations comprises:
generating a first sequence of acoustic sound signals at the external device, wherein the first sequence of acoustic signals represents a first portion of the at least one target virtual conversation and wherein the first sequence of acoustic sound signals is delivered to the recipient via the hearing device;
receiving, from the recipient, a spoken response to the first sequence of acoustic sound signals;
following receipt of the response to the first sequence of acoustic sound signals, iteratively generating one or more additional sequences of acoustic sound signals at the external device for delivery to the recipient via the hearing device; and
iteratively receiving additional spoken responses to each of the one or more additional sequences of acoustic sound signals delivered to the recipient via the hearing device,
wherein each of the one or more additional sequences of acoustic sound signals is dynamically generated based on an immediately preceding one of the additional spoken responses received from the recipient, and wherein each of the one or more additional sequences of acoustic sound signals represents a portion of the at least one target virtual conversation.

8. The one or more non-transitory computer readable storage media of claim 7, wherein the instructions operable to iteratively generate one or more additional sequences of acoustic sound signals at the external device for delivery to the recipient via the hearing device comprise instructions operable to at least one of:
dynamically generate at least one of the one or more additional sequences to include at least one clarification scenario; and
dynamically generate at least one of the one or more additional sequences to include at least one negotiation scenario.

9. The one or more non-transitory computer readable storage media of claim 1 or 2, wherein the instructions operable to deliver (267, 268, 269, 494) the one or more pre-recorded conversations to the recipient comprise instructions operable to at least one of:
- deliver at least one first pre-recorded conversation without background noise and subsequently deliver at least one second pre-recorded conversation with introduced background noise and/or distortion; and
- deliver at least one first pre-recorded conversation with a first number of conversational turns and subsequently deliver at least one second pre-recorded conversation with a second number of conversational turns, wherein the second number of conversational turns is greater than the first number of conversational turns.

10. The one or more non-transitory computer readable storage media of claim 1 or 2, wherein the instructions operable to deliver (267, 268, 269, 494) the one or more pre-recorded conversations to the recipient comprise instructions operable to:
deliver at least one first pre-recorded conversation; and
subsequently deliver at least one second pre-recorded conversation, wherein the at least second pre-recorded conversation newly introduces a clarification scenario or a negotiation scenario.

11. The one or more non-transitory computer readable storage media of claim 1 or 2, wherein the instructions operable to conduct (270, 271, 272, 496) a plurality of virtual conversations with the recipient comprise instructions operable to at least one of:
- conduct at least one first virtual conversation with a first number of conversational turns and subsequently conduct at least one second virtual conversation with a second number of conversational turns, wherein the second number of conversational turns is greater than the first number of conversational turns;
- conduct at least one first virtual conversation without background noise and subsequently conduct at least one second virtual conversation with introduced background noise;
- conduct at least one virtual conversation without distortion and subsequently conduct at least one second virtual conversation with introduced distortion;
- conduct at least one first virtual conversation and subsequently conduct at least one second virtual conversation, wherein the at least one second virtual conversation requires the recipient to verbalize one or more clarifications or one or more negotiations.

12. A system (305) for developing cognitive resources of a recipient of hearing device for telephone usage, comprising:
one or more network adapters (378) for communication with the hearing device:
a memory (376);
a user interface (379, 380); and
one or more processors (375) configured to:
train the recipient to extract, in real-time, content of received stimulation signals associated with a degraded signal quality associated with telephones and in the absence of visual cues; and
conduct a plurality of virtual conversations with the recipient, wherein the plurality of virtual conversations are conducted with increasing levels of listening and response complexity.

13. The system (305) of claim 12, wherein to train the recipient to extract, in real-time, content of received stimulation signals associated with a degraded signal quality associated with telephones and in the absence of visual cues, the one or more processors are configured for at least one of:
- delivering a plurality of sound signals to the recipient via the hearing device, wherein the plurality of sound signals represent a set of predetermined auditory targets; and
- delivering a plurality of pre-recorded conversations to the recipient to the recipient, where the plurality of pre-recorded conversations are delivered with increasing listening complexity.

14. The system of claim 12 or 13, wherein the memory (376) comprises one or more non-transitory computer readable storage media according to any one of claims 1 to 11.

## Patentansprüche

1. Ein oder mehrere nichtflüchtiges/nichtflüchtige, computerlesbares/computerlesbare Speichermedium/Speichermedien, das/die Befehle umfasst/umfassen, die, wenn sie von einem Prozessor einer externen Vorrichtung ausgeführt werden, den Prozessor veranlassen zum:
Ausgeben (266, 492) eines oder mehrerer vorgegebener Hör-Objekt/e an einen Träger einer Hörvorrichtung über die externe Vorrichtung und die Hörvorrichtung, wobei das eine oder die mehreren vorgegebenen Hör-Objekt/e Informationen umfasst/umfassen, die üblicherweise über ein Telefon verarbeitet werden;
Ausgeben (267, 268, 269, 494) eines oder mehrerer voraufgezeichneten/voraufgezeichneter Gesprächs/Gespräche an den Träger, über die externe Vorrichtung und die Hörvorrichtung; sowie
Führen (270, 271, 272, 496) eines oder mehrerer interaktiven/interaktiver, virtuellen/virtueller Gesprächs/Gespräche mit dem Träger über die externe Vorrichtung und die Hörvorrichtung.

2. Ein oder mehrere nichtflüchtiges/nichtflüchtige, computerlesbares/computerlesbare Speichermedium/Speichermedien nach Anspruch 1, wobei die Befehle, durch die in Funktion das eine oder die mehreren vorgegebene/n Hör-Objekt/e an den Träger ausgegeben werden kann/können (266, 492) Befehle umfassen, durch die in Funktion durchgeführt werden kann:
Erzeugen eines oder mehrerer Schallsignals/Signale an der externen Vorrichtung, wobei jedes von dem einen oder den mehreren Schallsignal/en ein vorgegebenes Hör-Objekt repräsentiert und jedes von dem einen oder den mehreren Schallsignal/en an den Träger über die von dem Träger verwendete Hörvorrichtung ausgegeben wird; sowie
Empfangen einer Angabe der Wahrnehmung jedes von dem einen oder den mehreren Schallsignal/en durch den Träger nach Ausgabe jedes von dem einen oder den mehreren Schallsignal/en an den Träger, insbesondere Empfangen der Angabe der Wahrnehmung des Trägers über eine Benutzerschnittstelle der externen Vorrichtung oder Empfangen einer gesprochenen Angabe der Wahrnehmung des Trägers über ein Mikrofon der externen Vorrichtung.

3. Ein oder mehrere nichtflüchtiges/nichtflüchtige, computerlesbares/computerlesbare Speichermedium/Speichermedien nach Anspruch 1 oder 2, wobei die Befehle, durch die in Funktion jedes von dem einen oder den mehreren voraufgezeichneten Gespräch/Gesprächen an den Träger über die Hörvorrichtung ausgegeben werden kann (267, 268, 269, 404), Befehle umfassen, durch die in Funktion durchgeführt werden kann:
Erzeugen einer Sequenz akustischer Schallsignale an der externen Vorrichtung, wobei jedes akustische Schallsignal ein zugehöriges voraufgezeichnetes Gespräch repräsentiert und wobei die Sequenz akustischer Schallsignale an den Träger über die Hörvorrichtung ausgegeben wird.

4. Ein oder mehrere nichtflüchtiges/nichtflüchtige, computerlesbares/computerlesbare Speichermedium/Speichermedien nach Anspruch 1 oder 2, das/die des Weiteren Befehle umfasst/umfassen, durch die in Funktion durchgeführt werden kann:
Abfragen des Trägers mit einer oder mehreren Frage/n bezüglich eines Inhaltes des vorangegangenen voraufgezeichneten Gesprächs nach Ausgabe jedes von dem einen oder den mehreren voraufgezeichneten Gespräch/en an den Träger über die Hörvorrichtung, wobei jede von dem einen oder den mehreren voraufgezeichneten Gespräch/en an einer Benutzerschnittstelle der externen Vorrichtung angezeigt wird; und
Empfangen einer Antwort auf jede von der einen oder den mehreren Frage/n von dem Träger, insbesondere Empfangen einer Benutzereingabe über eine oder mehrere Eingabevorrichtung/en der Benutzerschnittstelle oder Empfangen einer gesprochenen Antwort auf jede von der einen oder den mehreren Frage/n.

5. Ein oder mehrere nichtflüchtiges/nichtflüchtige, computerlesbares/computerlesbare Speichermedium/Speichermedien nach Anspruch 1 oder 2, wobei die Befehle, durch die in Funktion das eine oder die mehreren voraufgezeichneten Gespräch/Gespräche an den Träger über die Hörvorrichtung ausgegeben werden kann/können (267, 268, 269, 494), Befehle umfassen, durch die in Funktion durchgeführt werden kann:
Ausgeben (267) einer ersten Gruppe voraufgezeichneter Gespräche an den Träger über die Hörvorrichtung, wobei jedes der voraufgezeichneten Gespräche in der ersten Gruppe voraufgezeichneter Gespräche annähernd eine erste Anzahl von Gesprächswendungen sowie ein erstes Inhaltsniveau aufweist, die so ausgeführt sind, dass sich ein relevantes Schwellenniveau an Kompetenz entwickelt; und
Ausgeben (268) einer zweiten Gruppe voraufgezeichneter Gespräche an den Träger über die Hörvorrichtung nach Ausgabe (267) der ersten Gruppe voraufgezeichneter Gespräche, wobei jedes der voraufgezeichneten Gespräche in der zweiten Gruppe voraufgezeichneter Gespräche wenigstens eine zweite Anzahl von Gesprächswendungen aufweist, die größer ist als die erste Anzahl von Gesprächswendungen.

6. Ein oder mehrere nichtflüchtiges/nichtflüchtige, computerlesbares/computerlesbare Speichermedium/Speichermedien nach Anspruch 5, wobei die Befehle, durch die in Funktion das eine oder die mehreren voraufgezeichneten Gespräch/Gespräche an den Träger über die Hörvorrichtung ausgegeben werden kann/können (267, 268, 269, 494), Befehle umfassen, durch die in Funktion durchgeführt werden kann:
- Ausgeben einer dritten Gruppe voraufgezeichneter Gespräche an den Träger über die Hörvorrichtung nach Ausgabe (268) der zweiten Gruppe voraufgezeichneter Gespräche, wobei jedes der voraufgezeichneten Gespräche in der dritten Gruppe voraufgezeichneter Gespräche wenigstens eine dritte Anzahl von Gesprächswendungen aufweist, die größer ist als die zweite Anzahl von Gesprächswendungen; oder/und
- Ausgeben (269) einer dritten Gruppe voraufgezeichneter Gespräche an den Träger über die Hörvorrichtung nach Ausgabe (268) der zweiten Gruppe voraufgezeichneter Gespräche, wobei jedes der voraufgezeichneten Gespräche in der dritten Gruppe voraufgezeichneter Gespräche ein Verhandlungs-Szenario oder/und ein Klärungs-Szenario einschließt.

7. Ein oder mehrere nichtflüchtiges/nichtflüchtige, computerlesbares/computerlesbare Speichermedium/Speichermedien nach Anspruch 1 oder 2, wobei die Befehle, durch die in Funktion jedes von dem einen oder den mehreren interaktiven virtuellen Gespräch/en mit dem Träger geführt werden kann (270, 271, 272, 496) Befehle umfassen, durch die in Funktion ein oder mehrere virtuelle Ziel-Gespräch/e geführt werden kann/können, und wobei Führen wenigstens eines von dem einen oder den mehreren virtuellen Ziel-Gespräch/en umfasst:
Erzeugen einer ersten Sequenz akustischer Schallsignale an der externen Vorrichtung, wobei die erste Sequenz akustischer Signale einen ersten Abschnitt des wenigstens einen virtuellen Ziel-Gesprächs repräsentiert und die erste Sequenz akustischer Schallsignale an den Träger über die Hörvorrichtung ausgegeben wird;
Empfangen einer gesprochenen Antwort auf die erste Sequenz akustischer Schallsignale von dem Träger;
iteratives Erzeugen einer oder mehrerer zusätzlichen/zusätzlicher Sequenz/en akustischer Schallsignale an der externen Vorrichtung zur Ausgabe an den Träger über die Hörvorrichtung nach Empfang der Antwort auf die erste Sequenz akustischer Schallsignale; und
iteratives Empfangen zusätzlicher gesprochener Antworten auf jede von der einen oder den mehreren zusätzlichen Sequenz/en akustischer Schallsignale, die an den Träger über die Hörvorrichtung ausgegeben wird/werden,
wobei jede von der einen oder den mehreren zusätzlichen Sequenz/en akustischer Schallsignale dynamisch auf Basis einer unmittelbar vorangehenden der von dem Träger empfangenen zusätzlichen gesprochenen Antworten erzeugt wird, und wobei jede von der einen oder den mehreren zusätzlichen Sequenz/en akustischer Schallsignale einen Abschnitt des wenigstens einen virtuellen Ziel-Gesprächs repräsentiert.

8. Ein oder mehrere nichtflüchtiges/nichtflüchtige, computerlesbares/computerlesbare Speichermedium/Speichermedien nach Anspruch 7, wobei die Befehle, durch die iterativ eine oder mehrere zusätzliche/ Sequenz/en akustischer Schallsignale an der externen Vorrichtung zur Ausgabe an den Träger über die Hörvorrichtung erzeugt werden, Befehle umfassen, durch die durchgeführt werden kann:
dynamisches Erzeugen wenigstens einer von der einen oder den mehreren zusätzlichen Sequenz/en so, dass sie wenigstens ein Klärungs-Szenario einschließt/einschließen; oder/und
dynamisches Erzeugen wenigstens einer von der einen oder den mehreren zusätzlichen Sequenz/en so, dass sie wenigstens ein Verhandlungs-Szenario einschließt/einschließen.

9. Ein oder mehrere nichtflüchtiges/nichtflüchtige, computerlesbares/computerlesbare Speichermedium/Speichermedien nach Anspruch 1 oder 2, wobei die Befehle, durch die in Funktion das eine oder die mehreren voraufgezeichneten Gespräch/Gespräche an den Träger über die Hörvorrichtung ausgegeben werden kann/können (267, 268, 269, 494), Befehle umfassen, durch die in Funktion durchgeführt werden kann:
- Ausgeben wenigstens eines ersten voraufgezeichneten Gesprächs ohne Hintergrundgeräusch und anschließend Ausgeben wenigstens eines zweiten voraufgezeichneten Gesprächs mit eingefügtem Hintergrundgeräusch und/ oder Verzerrung; sowie
- Ausgeben wenigstens eines ersten voraufgezeichneten Gesprächs mit einer ersten Anzahl von Gesprächswendungen und anschließend Ausgeben wenigstens eines zweiten voraufgezeichneten Gesprächs mit einer zweiten Anzahl von Gesprächswendungen, wobei die zweite Anzahl von Gesprächswendungen größer ist als die erste Anzahl von Gesprächswendungen.

10. Ein oder mehrere nichtflüchtiges/nichtflüchtige, computerlesbares/computerlesbare Speichermedium/Speichermedien nach Anspruch 1 oder 2, wobei die Befehle, durch die in Funktion das eine oder die mehreren voraufgezeichneten Gespräch/Gespräche an den Träger ausgegeben werden kann/können (267, 268, 269, 494), Befehle umfassen, durch die in Funktion durchgeführt werden kann:
Ausgeben wenigstens eines ersten voraufgezeichneten Gesprächs; sowie
anschließend Ausgeben wenigstens eines zweiten voraufgezeichneten Gesprächs, wobei mit dem wenigstens einen zweiten voraufgezeichneten Gespräch ein Klärungs-Szenario oder ein Verhandlungs-Szenario neu eingeführt wird.

11. Ein oder mehrere nichtflüchtiges/nichtflüchtige, computerlesbares/computerlesbare Speichermedium/Speichermedien nach Anspruch 1 oder 2, wobei die Befehle, durch die in Funktion eine Vielzahl virtueller Gespräche mit dem Träger geführt werden kann (270, 271, 272, 496), Befehle umfassen, durch die in Funktion durchgeführt werden kann:
- Führen wenigstens eines ersten virtuellen Gesprächs mit einer ersten Anzahl von Gesprächswendungen und anschließend Führen wenigstens eines zweiten virtuellen Gesprächs mit einer zweiten Anzahl von Gesprächswendungen, wobei die zweite Anzahl von Gesprächswendungen größer ist als die erste Anzahl von Gesprächswendungen;
- Führen wenigstens eines ersten virtuellen Gesprächs ohne Hintergrundgeräusch und anschließend Führen wenigstens eines zweiten virtuellen Gesprächs mit eingefügter Hintergrundgeräusch;
- Führen wenigstens eines virtuellen Gesprächs ohne Verzerrung und anschließend Führen wenigstens eines zweiten virtuellen Gesprächs mit eingefügter Verzerrung; oder/und
- Führen wenigstens eines ersten virtuellen Gesprächs und anschließend Führen wenigstens eines zweiten virtuellen Gesprächs, wobei das wenigstens eine zweite virtuelle Gespräch erfordert, dass der Träger eine oder mehrere Klärung/en oder/eine oder mehrere Verhandlung/en verbalisiert.

12. System (305) zum Entwickeln kognitive Ressourcen eines Trägers einer Hörvorrichtung für Nutzung mittels Telefon, das umfasst:
einen oder mehrere Netzwerkadapter (378) für Kommunikation mit der Hörvorrichtung;
einen Speicher (376);
eine Benutzerschnittstelle (379, 380); sowie
einen oder mehrere Prozessor/en (375) der/die konfiguriert ist/sind zum:
Trainieren des Trägers zum Extrahieren von Inhalt empfangener Stimulationssignale, die mit einer verminderten Signalqualität in Verbindung mit Telefonen und dem Nichtvorhandensein visueller Hinweise zusammenhängen, in Echtzeit; sowie
Führen einer Vielzahl virtueller Gespräche mit dem Träger, wobei die Vielzahl virtueller Gespräche mit zunehmenden Niveaus an Komplexität beim Hören und antworten geführt werden.

13. System (305) nach Anspruch 12, wobei der eine oder die mehreren Prozessor/en, um den Träger zum Extrahieren von Inhalt empfangener Stimulationssignale, die mit einer verminderten Signalqualität in Verbindung mit Telefonen und dem Nichtvorhandensein visueller Hinweise zusammenhängen, in Echtzeit zu trainieren, konfiguriert ist/sind zum:
- Ausgeben einer Vielzahl von Schallsignalen an den Träger über die Hörvorrichtung, wobei die Vielzahl von Schallsignalen eine Gruppe vorgegebener Hör-Objekte repräsentieren; oder/und
- Ausgeben einer Vielzahl voraufgezeichneter Gespräche mit dem Empfänger an den Empfänger, wobei die Vielzahl voraufgezeichneter Gespräche mit zunehmender Komplexität beim Hören ausgegeben werden.

14. System nach Anspruch 12 oder 13, wobei der Speicher (376) ein oder mehrere nichtflüchtiges/nichtflüchtige, computerlesbares/computerlesbare Speichermedium/Speichermedien nach einem der Ansprüche 1 bis 11 umfasst.

## Revendications

1. Un ou plusieurs supports de stockage non transitoires lisibles par ordinateur comprenant des instructions qui, lorsqu'elles sont exécutées par un processeur d'un dispositif externe, amènent le processeur à :
délivrer (266, 492), via le dispositif externe et un dispositif auditif, une ou plusieurs cibles audio prédéterminées à un bénéficiaire de l'appareil auditif, dans lesquels les une ou plusieurs cibles audio prédéterminées comprennent des informations typiquement traitées via un téléphone ;
délivrer (267, 268, 269, 494), via le dispositif externe et le dispositif auditif, une ou plusieurs conversations préenregistrées au bénéficiaire ; et
mener (270, 271, 272, 496), via le dispositif externe et le dispositif auditif, une ou plusieurs conversations virtuelles interactives avec le bénéficiaire.

2. Un ou plusieurs supports de stockage non transitoires lisibles par ordinateur selon la revendication 1, dans lesquels les instructions opérationnelles pour délivrer (266, 492) les une ou plusieurs cibles audio prédéterminées au bénéficiaire comprennent des instructions opérationnelles pour :
générer un ou plusieurs signaux sonores au niveau du dispositif externe, dans lesquels chacun des un ou plusieurs signaux sonores représente une cible audio prédéterminée et dans lesquels chacun des un ou plusieurs signaux sonores est délivré au bénéficiaire via le dispositif auditif utilisé par le bénéficiaire ; et
après avoir délivré chacun des un ou plusieurs signaux sonores au bénéficiaire, recevoir une indication de la perception du bénéficiaire de chacun des un ou plusieurs signaux sonores, en particulier recevoir l'indication de la perception du bénéficiaire via une interface utilisateur du dispositif externe, ou recevoir une indication vocale de la perception du bénéficiaire via un microphone du dispositif externe.

3. Un ou plusieurs supports de stockage non transitoires lisibles par ordinateur selon la revendication 1 ou 2, dans lesquels les instructions opérationnelles pour délivrer (267, 268, 269, 404) chacune des une ou plusieurs conversations préenregistrées au bénéficiaire via le dispositif auditif comprennent des instructions opérationnelles pour : générer une séquence de signaux sonores acoustiques au niveau du dispositif externe, dans lesquels chaque signal sonore acoustique représente une conversation préenregistrée associée et dans lesquels la séquence de signaux sonores acoustiques est délivrée au bénéficiaire via le dispositif auditif.

4. Un ou plusieurs supports de stockage non transitoires lisibles par ordinateur selon la revendication 1 ou 2, comprenant en outre des instructions opérationnelles pour :
après avoir délivré chacune des une ou plusieurs conversations préenregistrées au bénéficiaire via le dispositif auditif, interroger le bénéficiaire avec une ou plusieurs questions concernant un contenu d'une conversation préenregistrée précédente, dans lesquels chacune des une ou plusieurs questions est affichée sur une interface utilisateur du dispositif externe ; et
recevoir, du bénéficiaire, une réponse à chacune à chacune des une ou plusieurs questions, recevoir en particulier une entrée utilisateur via un ou plusieurs dispositifs d'entrée de l'interface utilisateur ou recevoir une réponse vocale à chacune des une ou plusieurs questions.

5. Un ou plusieurs supports de stockage non transitoires lisibles par ordinateur selon la revendication 1 ou 2, dans lesquels les instructions opérationnelles pour délivrer (267, 268, 269, 494) les une ou plusieurs conversations préenregistrées au bénéficiaire via le dispositif auditif comprennent des instructions opérationnelles pour :
délivrer (267) un premier ensemble de conversations préenregistrées au bénéficiaire via le dispositif auditif, dans lesquels chacune des conversations préenregistrées du premier ensemble de conversations préenregistrées comporte approximativement un premier nombre de prises de parole conversationnelles et un premier niveau de contenu configuré pour développer un niveau de seuil de compétence pertinent ; et
après avoir délivré (267) le premier ensemble de conversations préenregistrées, délivrer (268) un deuxième ensemble de conversations préenregistrées au bénéficiaire via le dispositif auditif, dans lesquels chacune des conversations préenregistrées du deuxième ensemble de conversations préenregistrées comporte au moins un deuxième nombre de prises de parole conversationnelles qui est supérieur au premier nombre de prises de parole conversationnelles.

6. Un ou plusieurs supports de stockage non transitoires lisibles par ordinateur selon la revendication 5, dans lesquels les instructions opérationnelles pour délivrer (267, 268, 269, 494) les une ou plusieurs conversations préenregistrées au bénéficiaire via le dispositif auditif comprennent en outre des instructions opérationnelles pour :
- après avoir délivré (268) le deuxième ensemble de conversations préenregistrées, délivrer un troisième ensemble de conversations préenregistrées au bénéficiaire via le dispositif auditif, dans lesquels chacune des conversations préenregistrées du troisième ensemble de conversations préenregistrées comporte au moins un troisième nombre de prises de parole conversationnelles qui est supérieur au deuxième nombre de prises de parole conversationnelles ; et/ou
- après avoir délivré (268) le deuxième ensemble de conversations préenregistrées, délivrer (269) un troisième ensemble de conversations préenregistrées au bénéficiaire via le dispositif auditif, dans lesquels
chacune des conversations préenregistrées du troisième ensemble de conversations préenregistrées comporte au moins un parmi un scénario de négociation et un scénario de clarification.

7. Un ou plusieurs supports de stockage non transitoires lisibles par ordinateur selon la revendication 1 ou 2, dans lesquels les instructions opérationnelles pour mener (270, 271, 272, 496) chacune des une ou plusieurs conversations virtuelles interactives avec le bénéficiaire comprennent des instructions opérationnelles pour mener une ou plusieurs conversations virtuelles cibles, et dans lesquels la conduite d'au moins une des une ou plusieurs conversations virtuelles cibles comprend les étapes consistant à :
générer une première séquence de signaux sonores acoustiques au niveau du dispositif externe, dans lesquels la première séquence de signaux acoustiques représente une première partie de la au moins une conversation virtuelle cible et dans lesquels la première séquence de signaux sonores acoustiques est délivrée au bénéficiaire via le dispositif auditif ;
recevoir, du bénéficiaire, une réponse vocale à la première séquence de signaux sonores acoustiques ;
après réception de la réponse à la première séquence de signaux sonores acoustiques, générer de manière itérative une ou plusieurs séquences supplémentaires de signaux sonores acoustiques au niveau du dispositif externe pour les délivrer au bénéficiaire via le dispositif auditif ; et
recevoir de manière itérative des réponses vocales supplémentaires à chacune des une ou plusieurs séquences supplémentaires de signaux sonores acoustiques délivrées au bénéficiaire via le dispositif auditif,
dans lesquels chacune des une ou plusieurs séquences supplémentaires de signaux sonores acoustiques est générée dynamiquement sur la base d'une réponse immédiatement précédente parmi les réponses vocales supplémentaires reçues du bénéficiaire, et dans lesquels chacune des une ou plusieurs séquences supplémentaires de signaux sonores acoustiques représente une partie de la au moins une conversation virtuelle cible.

8. Un ou plusieurs supports de stockage non transitoires lisibles par ordinateur selon la revendication 7, dans lesquels les instructions opérationnelles pour générer itérativement une ou plusieurs séquences supplémentaires de signaux sonores acoustiques au niveau du dispositif externe pour les délivrer au bénéficiaire via le dispositif auditif, comprennent des instructions opérationnelles pour au moins :
générer de manière dynamique au moins une des une ou plusieurs séquences supplémentaires pour l'intégrer au niveau du au moins un scénario de clarification ; et/ou
générer de manière dynamique au moins une des une ou plusieurs séquences supplémentaires pour l'intégrer au niveau du au moins un scénario de négociation.

9. Un ou plusieurs supports de stockage non transitoires lisibles par ordinateur selon la revendication 1 ou 2, dans lesquels les instructions opérationnelles pour délivrer (267, 268, 269, 494) les une ou plusieurs conversations préenregistrées au bénéficiaire comprennent des instructions opérationnelles pour au moins :
- délivrer au moins une première conversation préenregistrée sans bruit de fond et par la suite, délivrer au moins une seconde conversation préenregistrée avec un bruit de fond et/ou une distorsion introduits ; et/ou
- délivrer au moins une première conversation préenregistrée avec un premier nombre de prises de parole conversationnelles et par la suite, délivrer au moins une seconde conversation préenregistrée avec un deuxième nombre de prises de parole conversationnelles, dans lesquels le deuxième nombre de prises de parole conversationnelles est supérieur au premier nombre de prises de parole conversationnelles.

10. Un ou plusieurs supports de stockage non transitoires lisibles par ordinateur selon la revendication 1 ou 2, dans lesquels les instructions opérationnelles pour délivrer (267, 268, 269, 494) les une ou plusieurs conversations préenregistrées au bénéficiaire comprennent des instructions opérationnelles pour :
délivrer au moins une première conversation préenregistrée ; et
délivrer par la suite au moins une seconde conversation préenregistrée, dans lesquels la au moins une seconde conversation préenregistrée introduit nouvellement un scénario de clarification ou un scénario de négociation.

11. Un ou plusieurs supports de stockage non transitoires lisibles par ordinateur selon la revendication 1 ou 2, dans lesquels les instructions opérationnelles pour mener (270, 271, 272, 496) une pluralité de conversations virtuelles avec le bénéficiaire comprennent des instructions opérationnelles pour au moins une opération parmi :
- la conduite d'au moins une première conversation virtuelle avec un premier nombre de prises de parole conversationnelles et par la suite la conduite d'au moins une seconde conversation virtuelle avec un deuxième nombre de prises de parole conversationnelles, dans lesquels le deuxième nombre de prises de parole conversationnelles est supérieur au premier nombre de prises de parole conversationnelles ;
- la conduite d'au moins une première conversation virtuelle sans bruit de fond et par la suite la conduite d'au moins une seconde conversation virtuelle avec un bruit de fond introduit ;
- la conduite d'au moins une conversation virtuelle sans distorsion et par la suite la conduite d'au moins une seconde conversation virtuelle avec une distorsion introduite ;
- la conduite d'au moins une première conversation virtuelle et par la suite la conduite d'au moins une seconde conversation virtuelle, dans lesquels la au moins une seconde conversation virtuelle exige que le bénéficiaire verbalise une ou plusieurs clarifications ou une ou plusieurs négociations.

12. Système (305) pour développer des ressources cognitives d'un bénéficiaire de dispositif auditif pour une utilisation téléphonique, comprenant :
un ou plusieurs adaptateurs réseau (378) pour communication avec le dispositif auditif ;
une mémoire (376) ;
une interface utilisateur (379, 380) ; et
un ou plusieurs processeurs (375) configurés pour :
entraîner le bénéficiaire à extraire, en temps réel, un contenu des signaux de stimulation reçus associés à une qualité de signal dégradée associée à des téléphones et en l'absence d'indices visuels ; et
mener une pluralité de conversations virtuelles avec le bénéficiaire, dans lequel la pluralité de conversations virtuelles est menée avec des niveaux croissants de complexité d'écoute et de réponse.

13. Système (305) selon la revendication 12, dans lequel pour entraîner le bénéficiaire à extraire, en temps réel, un contenu de signaux de stimulation reçus associés à une qualité de signal dégradée associée à des téléphones et, en l'absence d'indices visuels, les un ou plusieurs processeurs sont configurés pour au moins :
- délivrer une pluralité de signaux sonores au bénéficiaire via le dispositif auditif, dans lequel la pluralité de signaux sonores représente un ensemble de cibles audio prédéterminées ; et
- délivrer une pluralité de conversations préenregistrées au bénéficiaire, où la pluralité de conversations préenregistrées sont délivrées avec une complexité d'écoute croissante.

14. Système selon la revendication 12 ou 13, dans lequel la mémoire (376) comprend un ou plusieurs supports de stockage non transitoires lisibles par ordinateur selon l'une quelconque des revendications 1 à 11.
